# EUROPEAN PATENT APPLICATION

(11) **EP 1 279 376 A2**
(43) Date of publication of application: **29.01.2003**
(21) Application number: 02016617.9
(22) Date of filing: 25.07.2002
(51) Int. Cl.: A61B 19/00

(54) **Device and procedure for computer-assisted microsurgery**

(30) Priority: 27.07.2001 IT MI20011635
(71) Applicant: G.D.S. Giorgi Dynamic Stereotaxy S.r.l., 05100 Terni (IT)
(72) Inventor: Giorgi, Cesare, 20121 Milano (IT); Cossu, Antonio, 20121 Milano (IT)
(74) Representative: Rapisardi, Mariacristina

(57) **Abstract**

The device for computer-assisted microsurgery comprises at least one support for a plurality of sensors, suitable for detecting the position at least of fixed and removable localisers (6, 11), connected to a data-processor (4) in turn connected to a video (5) which indicates the position of fixed and removable localiser means (6, 11).

The sensors (3) are at least three in number and the processor (4) compares the data detected by the three sensors (3).

The procedure for computer-assisted microsurgery comprises the steps of acquiring first coordinates of removable localisers (11) in a first reference system, of acquiring, by detecting three coordinates, second coordinates of the removable localisers (11) with respect to a second reference system integral with a fixed localiser (6), of associating the first coordinates with the second coordinates determining the position of the removable localisers (11) in the second reference system.

## Description

The present invention refers to a device and procedure for computer-assisted microsurgery.

The devices for computer assisted microsurgery which currently exist comprise a pair of video cameras which are fixed onto fixed supports and which frame the operating region, connected to an electronic computer and to a viewing screen.

Conventional devices comprise a fixed localiser, consisting of a frame which carries a plurality of elements which can be detected by video cameras, which is made integral with the head of the patient during the operation since it is connected to the Mayfield headrest.

Conventional devices also comprise a detection pen, consisting of a pen which carries at one end an element which can be detected by video cameras, which is manipulated by the surgeon.

Moreover, the devices comprise a plurality of fiducials (elements which can be detected in a CAT scan or the like), which can be applied to the head of the patient before intervention.

Usually, before carrying out an operation, the fiducials are applied to the head of the patient (even the day before) and an examination is carried out which allows information on the inner anatomy of the patient to be gathered.

In particular such examinations can consist of a CAT (computer-assisted tomography) scan, or else magnetic resonance scanning, or another type of examination.

Fiducials contain a marking liquid which can be detected in the CAT scan and, therefore, the inner anatomy of the patient is determined with respect to a reference system in which the position of the fiducial is known.

Then, at the time of the operation, the patient is positioned in the operation zone in the visual field of the video cameras.

Beforehand, the fixed localiser is applied (made integral with the head of the patient) and one proceeds to set the video cameras to communicate to the computer the coordinates of the fiducials with respect to the fixed localiser.

The setting takes place by moving the detection pen close to each predetermined fiducial (upon computer request) so that the video cameras can acquire the position of every fiducial which is registered by the computer.

In this way, since the anatomy of the patient had already been detected with reference to the fiducials (during the CAT scan), and since the position of the fiducials is known with respect to the fixed localiser, the computer is able to represent the position of the detection pen in the reconstruction in 3 dimensions (3D) in relation to the head of the patient and, therefore, in relation to the fixed localiser.

Nevertheless, conventional devices of the type indicated have numerous drawbacks, due to the fact that the setting step, which is very important for a successful outcome of the surgical intervention, requires a lot of time.

Indeed, the detection pen has to be moved close to each fiducial and the position of the fiducial must be acquired.

Moreover, the pressure of the detection pen on the fiducials alters their geometry causing an error in the determination of the position.

Moreover, since conventional devices work by using just two video cameras simultaneously, in some cases there is an ambiguity in the acquisition of information (images).

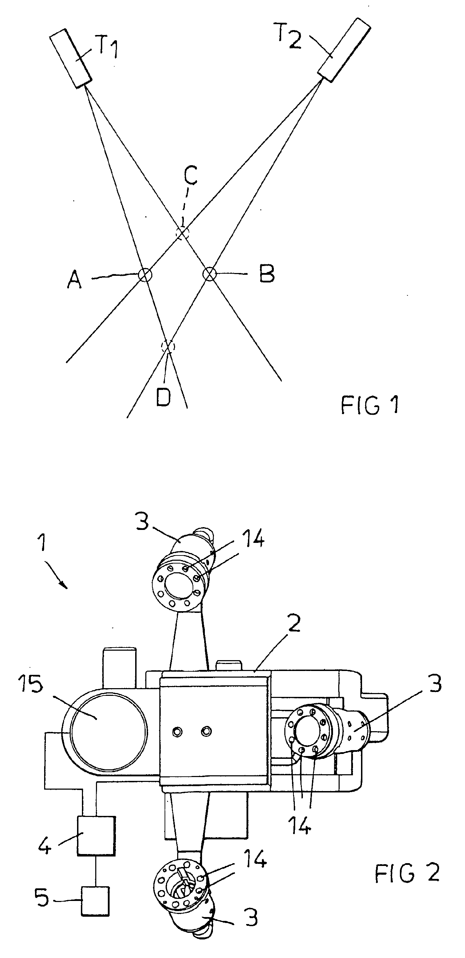

Indeed, as is clear from figure 1, if two video cameras T1, T2 frame points A,B, one is not able to check if the real points are A, B or else C, D; to solve the ambiguity in the acquisition of such information it is necessary to make use of auxiliary instruments (detection pen).

Moreover, in conventional devices in which the video cameras which are used to detect the position of the fiducials are situated far away from the patient and are fitted onto a fixed support, sometimes the visual field is blocked by the surgeon or by his assistants.

A further drawback of conventional devices is that to check the position where one is operating (for example where a portion of the skull is being removed) it is necessary to interrupt the operation and draw the removable localiser near to the portion involved in the operation.

The technical task proposed of the present invention is, therefore, that of eliminating the aforementioned technical drawbacks of the prior art.

In this technical task, a purpose of the invention is that of realising a device the use of which is not blocked by the presence of the surgeon or his assistants who can (involuntarily) prevent the video cameras from viewing the zone in which the operation is being performed.

In particular, the present invention allows to avoid it being necessary to approach a removable localiser to every fiducial, but allows acquisition to be carried out in a completely automatic manner.

Another purpose of the invention is that of realising a device which works using at least three video cameras simultaneously and which, therefore, is capable of detecting the position of points A, B with no uncertainty.

A further purpose of the invention is that of realising a device and a procedure for computer-assisted microsurgery which allow the registering step to be carried out in shorter spaces of time.

The last but not least purpose of the invention is that of realising a device and a procedure which allow the position (on the head) where one is operating to be checked without needing to interrupt the operation (to position the detection pen).

Appropriately, on the other hand, through the device and the procedure according to the invention, the position where one is operating is continually signalled (on the screen) and updated.

The technical task, as well as these and other tasks, according to the present invention are achieved by realising a device for computer-assisted microsurgery comprising at least one support for a plurality of sensors, suitable for detecting the position at least of localising means, connected to data processing means in turn connecting to means for indicating the position of said localising means, characterised in that said sensors are at least three in number, and in that said processing means compare the data detected by said at least three sensors.

The present invention also refers to a procedure for computer-assisted microsurgery, characterised in that it comprises the steps of acquiring first coordinates of removable localisers in a first reference system, of acquiring, by detecting three coordinates, second coordinates of the removable localisers with respect to a second reference system integral with a fixed localiser, of associating the first coordinates with the second coordinates determining the position at least of the removable localisers in the second reference system.

Other characteristics of the present invention are defined, moreover, in the subsequent claims.

Further characteristics and advantages of the invention shall become clearer from the description of a preferred but not exclusive embodiment of the device and of the procedure for computer-assisted microsurgery according to the present finding, where the device is illustrated for indicating and not limiting purposes in the attached drawings, in which:
- figure 1 shows a schematic view of two video cameras which frame two removable localiser elements;
- figure 2 shows a view from below of a portion of the device according to the finding;

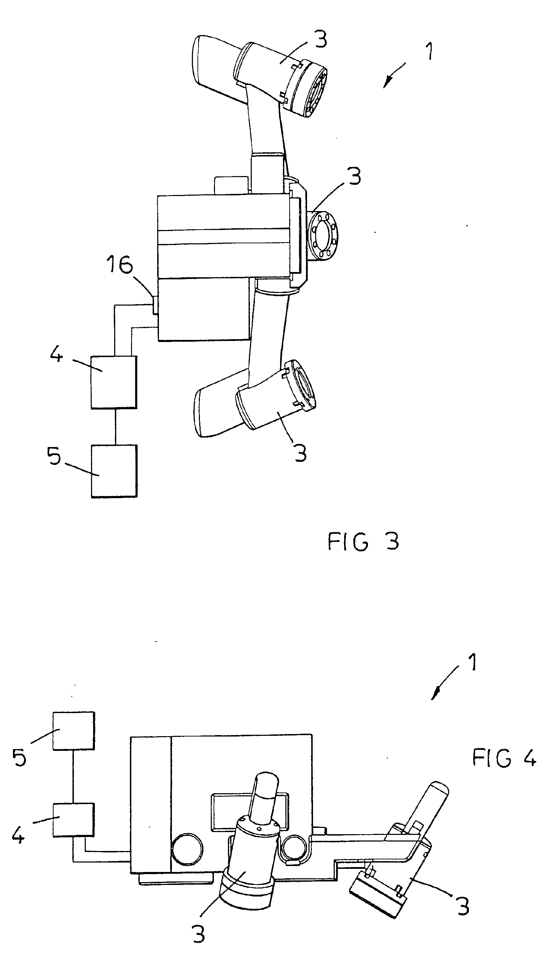

- figure 3 shows a front view of the device according to the finding;
- figure 4 shows a side view of the device according to the finding;

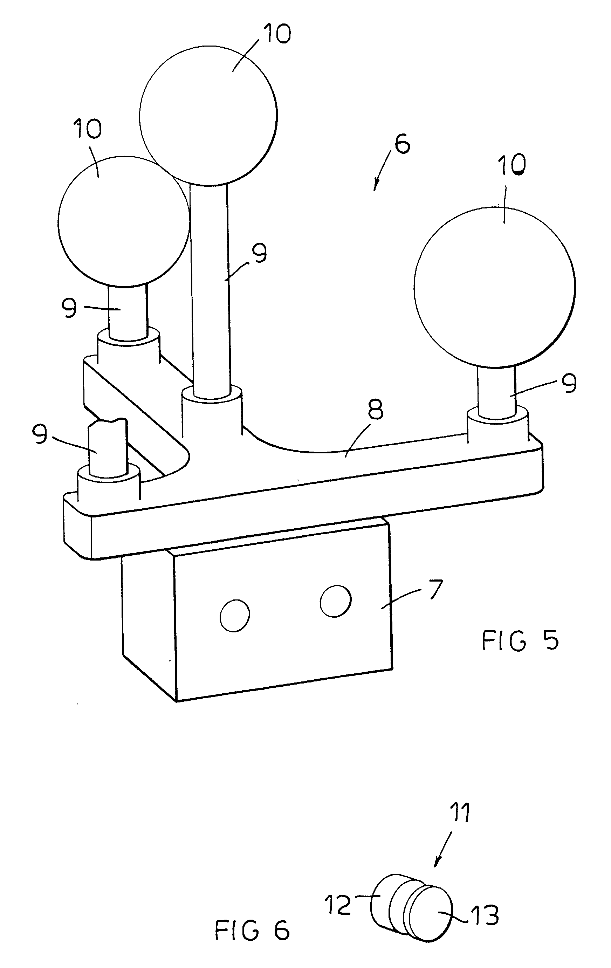

- figure 5 shows a perspective view of a fixed localiser of the device according to the finding;
- figure 6 shows a perspective view of a removable localiser or fiducial according to the finding;
- figure 7 shows a perspective view of a portion of a calibration apparatus; and
- figure 8 shows a scale front view of a plate of the apparatus of figure 7.

With reference to the quoted figures, a device for computer-assisted microsurgery is shown wholly indicated with reference numeral 1.

The device 1 comprises a support 2 for a plurality of sensors 3 suitable for detecting the position of localiser means 6, 11.

The sensors 3 are connected to data-processing means 4 which, in turn, are connected to indicator means 5 of the position of the localiser means 6, 11, such as a video screen.

Advantageously, the sensors 3 are at least three in number and the data processing means 4 compare the data detected by the three sensors.

Moreover, the device 1 comprises a fixed localiser 6, as localiser means, which is made integral with the patient during the surgical operation.

The fixed localiser 6 comprises, for example, an attachment element 7 which carries a substantially T-shaped base 8.

The base 8, in turn, carries four elongated elements 9 which each support a spherical element made of retro-reflecting material 10 to reflect electromagnetic radiation.

The device 1 also comprises a plurality of removable localisers or fiducials 11 as localiser means, realised through an airtight hollow body filled with a marking liquid which can be detected in a CAT or MR (magnetic resonance) scan.

Fiducials, which can be applied to the head of the patient, can also be detected by the video cameras, because they have a removable portion 13 made of retro-reflecting material which can be fixed to the hollow body.

The sensors 3 can be of whatever type but, in a preferred embodiment, comprise video cameras equipped with infrared radiation filters.

In this way the device works only using radiation in the infrared field, limiting disturbance due to light in the visible range.

The video cameras 3, moreover, support a plurality of infrared radiation emitters; in the example shown every video camera carries eight infrared emitting diodes.

Such emitters 14 have their axis (according to which the infrared radiation is emitted) substantially parallel to an axis of the video cameras 3.

The support 2 and the video cameras 3 are preferably fixed to an operating microscope 15, for example of the type equipped with a parallel output gate 16 suitable for transmitting information on the focusing and/or zooming of the microscope 15.

The invention also refers to a procedure for computer-assisted microsurgery.

The procedure for computer-assisted microsurgery according to the present invention comprises the steps of acquiring first coordinates of the removable localisers or fiducials 11 in a first reference system.

This is carried out, for example, through a CAT or MR (magnetic resonance) scan.

Then second coordinates of the removable localisers or fiducials with respect to a second reference system integral with a fixed localiser are acquired, detecting three coordinates.

This is carried out by using the video cameras, which detect infrared radiation reflected by the elements 10 of the fixed localiser and/or by the fiducials 13.

Then the first coordinates of the fiducials 11 are associated with the second coordinates (with respect to the fixed localiser) determining the position of the removable localisers in the second reference system.

Then, since the internal anatomy of the patient was determined in the first reference system (with the CAT or MR scan), the internal anatomy of the head of the patient is also determined with reference to the reference system of the fixed localiser 6.

The removable localisers 11 are taken away after the association between the first and second coordinates of the removable localisers or fiducials 11 has been carried out, since they are no longer necessary given that their position in the reference system of the fixed localiser has been identified.

Advantageously, the step of associating the first positions with the second positions is carried out by comparing the distances between the removable localisers or fiducials next to each other in each of the reference systems and by assigning the correspondence between the positions detected in the first and second reference system when the distance between two removable localisers or fiducials in the two reference systems is substantially the same.

Preferably, the correspondence is reached not only when such a distance is equal, but also when it is less than a predetermined margin.

In this way a margin is allowed for, which guarantees recognition even when the two values (for example due to calculations) move apart by very small amounts.

Moreover, in an appropriate manner, the electronic processor is also supplied with the information indicating the position of the optical axis of the microscope, which can thus be indicated or represented on the video with respect to the second reference system (of the fixed localiser).

In particular, this is carried out in an initial calibration step, in which the position of the optical axis with respect to the video cameras is memorised.

The electronic processor is also supplied with the information relative to the focusing and the zooming of the microscope, so as to find out in a known manner the depth of the point which is being looked at in the reconstruction.

The operation of the device for computer assisted microsurgery according to the invention can be clearly seen from that which has been described and illustrated and, in particular, is substantially the following.

Beforehand, the fiducials 11 are fixed onto the head of the patient (even the day before) and the scan (for example CAT or MR scan) is carried out.

Then (for example the following day) the portions 13 made of retro-reflecting material of the fiducials are applied to the body 12, the head of the patient is fixed onto the Mayfield headrest and the fixed localiser 6 is connected to the same Mayfield headrest or to its support.

In this way the fixed localiser 6 is integral with the patient's head.

Then the device is registered.

The registration is carried out by passing the microscope 15 over the head of the patient so that the video cameras 3 "see" the fiducials 11.

In practice, the emitter diodes emit infrared radiation which is reflected by the elements 10 of the fixed localiser or else by the fiducials 11.

The electronic processor 4 constitutes a reference system integral with the fixed localiser 6 and, therefore, determines the position of the fiducials 11 with respect to such a reference system.

Therefore, through the procedure which is also object of the invention, the association of the fiducials 11 in the two reference systems is carried out, i.e. a fiducial 11 which was detected during the CAT or MR scan is associated with every fiducial 11 which has been detected by the video cameras.

In this way it is possible to navigate in the internal anatomy of the head of the patient (which has been detected during the CAT or MR scan).

In practice, therefore, it is possible to represent the position in space of the optical axis of the microscope with respect to the internal anatomy of the head.

The electronic processor 4 always knows the position of the optical axis of the microscope 15, because the support 2 of the video cameras 3 is fixed onto the microscope 15.

Therefore, the electronic processor 4 also sends to the video 5 the information relative to the position and the orientation of the optical axis of the microscope 15, which can be represented, for example, as a line on the video.

The visualisation of the optical axis of the microscope 15 allows the position (and/or the depth) on which one is operating to be verified without it being necessary to interrupt the surgical intervention to do this.

Moreover, when the microscope 15 is suitable for providing data on its focusing and zooming, the processor, in a known manner, is also capable of processing such signals to work out the depth at which one is operating.

In practice, therefore, the device according to the finding is suitable for representing both the head of the patient and/or its internal anatomy, and the optical axis of the microscope on video; therefore, the surgeon can use the information which it provides him with in a very profitable manner.

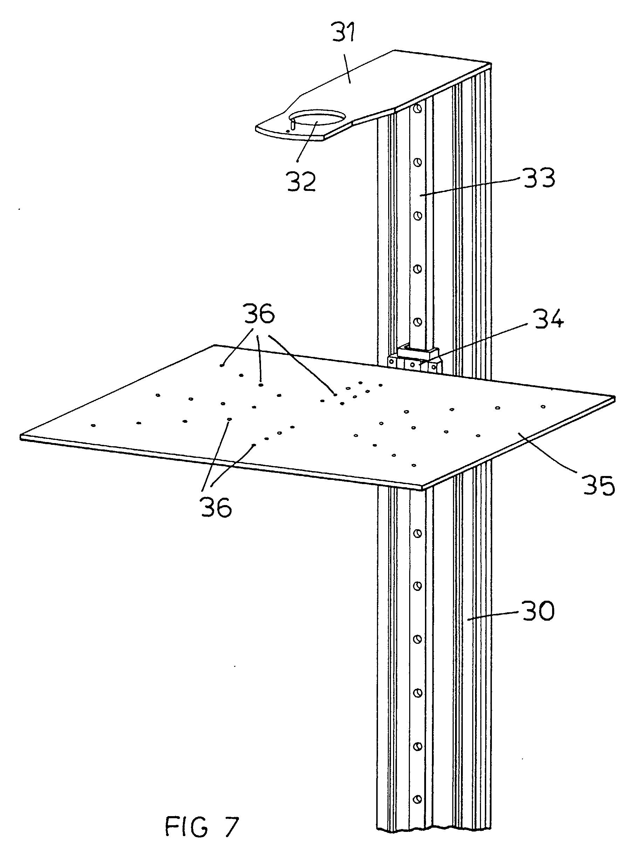

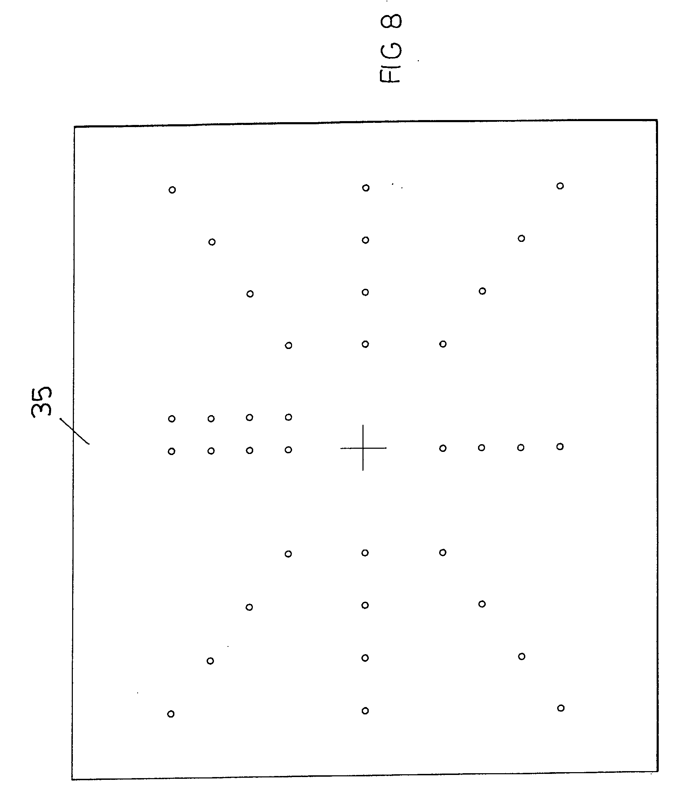

The calibration is carried out through the apparatus shown in figures 7 and 8.

The apparatus comprises a column 30 which carries at one end a support foot (not shown) and at the other end a support 31 for a microscope.

The support 30 has a through opening 32 where the microscope is attached.

The column 30, moreover, carries a guide 33 on which a trolley 34 slides driven in translation through a lever (not shown) suitable for giving the trolley 34 a micrometric displacement.

The trolley 34 carries a plate 35 (represented in scale in figure 8 - dimensions 450x500 millimetres) which supports a plurality of spherical retro-reflecting elements 36 arranged according to a predetermined geometry (shown in scale in the attached figure), where the position of the retro-reflecting elements is memorised in an electronic processor.

Such a device is used to calibrate the video cameras.

Indeed, when the video cameras are applied to the device 1 the microscope is faced towards the opening 32, with the optical axis which is substantially addressed towards the centre of the plate 35.

The calibration is carried out through a series of measurements (usually 4 or else 5) in which the plate 35 is positioned at a predetermined distance from the lens of the microscope and, then, the plate is displaced by a known amount.

In this way the processor, which receives the images from the video cameras and the information on the displacement of the plate 35, in a known way works out the roto-translation matrices (calibration parameters) which allow the three coordinates in space to be worked out from the images detected by the video cameras.

In the same way, the processor is capable of detecting and memorising the optical axis of the microscope 15 with respect to the video cameras (which substantially coincides with the vertical axis of the calibration apparatus).

The calibration apparatus also allows the correspondence between the focusing distance and the distance between the microscope and the position which is observed with the microscope to be determined.

For such a purpose a series of measurements are carried out with which a map is worked out which associates every different focal position with a determined lens distance (microscope) - observed position.

In practice, it has been noted how the device and the procedure for computer-assisted microsurgery according to the invention are particularly advantageous because they allow the ambiguities in the detection of the fiducials by the video cameras to be eliminated and, moreover, make the patient registering step, which must necessarily be carried out before every intervention, particularly simple and fast.

Moreover, by using the device according to the present invention it is no longer necessary to use the detection pen.

The device and the procedure for computer-assisted microsurgery thus conceived are susceptible to numerous modifications and variants, all covered by the inventive concept; moreover, all of the details can be replaced with technically equivalent elements.

In practice, the materials used, as well as the sizes, can be whatever according to the requirements and the state of the art.

## Claims

1. Device (1) for computer-assisted microsurgery comprising at least one support (2) for a plurality of sensors (3), suitable for detecting the position at least of localiser means (6, 11), connected to data-processing means (4) in turn connected to indicator means (5) of the position of said localiser means (6, 11), **characterised in that** said sensors (3) are at least two in number, and **in that** said processing means (4) compare the data detected by said at least three sensors (3).

2. Device (1) according to claim 1, **characterised in that** said sensors (3) are at least three in number.

3. Device (1) according to one or more of the previous claims, **characterised in that** it does not comprise a detection pen, and **in that** it allows the position upon which one is operating to be verified without it being necessary to interrupt the surgical intervention to do this.

4. Device (1) according to one or more of the previous claims, **characterised in that** said localiser means (6, 11) comprise at least one fixed localiser (6), suitable for being made integral with the patient during the surgical operation.

5. Device (1) according to one or more of the previous claims, **characterised in that** said localiser means (6, 11) comprise a plurality of removable localisers or fiducials (11), which can be detected both by a CAT or MR scan and by said sensors (3) and which are suitable for being applied to a patient.

6. Device (1) according to one or more of the previous claims, **characterised in that** said sensors (3) comprise video cameras.

7. Device (1) according to one or more of the previous claims, **characterised in that** said video cameras (3) comprise infrared radiation filters.

8. Device (1) according to one or more of the previous claims, **characterised in that** said video cameras (3) support a plurality of infra-red radiation emitters (14), said emitters (14) having their axis substantially parallel to an axis of said video cameras.

9. Device (1) according to one or more of the previous claims, **characterised in that** said support (2) and/or said video cameras (3) are connected to an operating microscope (15).

10. Device (1) according to one or more of the previous claims, **characterised in that** said operating microscope (15) has an output gate (16) suitable for transmitting information on it focusing and zooming.

11. Device (1) according to one or more of the previous claims, **characterised in that** said removable localisers (11) comprise a body (12) to which a portion (13) which can be detected by said video cameras (3) is connected.

12. Device (1) according to one or more of the previous claims, **characterised in that** said portion (13) which can be detected by said video cameras (3) is removably connected to said body (12).

13. Procedure for computer-assisted microsurgery, **characterised in that** it comprises the steps of acquiring first coordinates of removable localisers (11) in a first reference system, of acquiring, by detecting three coordinates, second coordinates of the removable localisers (11) with respect to a second reference system integral with a fixed localiser (6), of associating the first coordinates with the second coordinates determining the position of at least the removable localisers (11) in the second reference system.

14. Procedure according to claim 13, **characterised in that** when the positions of the removable localisers (11) are acquired the internal anatomy of the patient is also acquired, and **in that** from the position of the removable localisers in the second reference system the internal anatomy of the patient in the second reference system is worked out.

15. Procedure according to claim 13 or 14, **characterised in that** the removable localisers (11) are taken away after the association between the first and second coordinates has been carried out.

16. Procedure according to one or more of claims 13 to 15, **characterised in that** the step of associating the first coordinates with the second coordinates is carried out by comparing the distances between removable localisers (11) next to each other in each of the reference systems and by assigning the correspondence between the positions detected in the first and second reference system when the distances between two removable localisers (11) in the two reference systems are substantially the same.

17. Procedure according to one or more of claims 12 to 16, **characterised in that** it provides the information indicating the position of the optical axis of the microscope (15), to indicate it in said second reference system.

18. Procedure according to one or more of claims 13 to 17, **characterised in that** it provides the information relative to the focusing and zooming of the microscope (15), to work out the depth at which one is operating.

19. Calibration apparatus (30), **characterised in that** it comprises a plate (35) which supports a plurality of retro-reflecting elements (36) arranged according to a predetermined geometry, where the position of the retro-reflecting elements (36) is memorised in an electronic processor.

20. Calibration procedure, **characterised in that** it carries out a series of measurements in which a plate (35) is positioned, which supports a plurality of retro-reflecting elements (36) arranged according to a predetermined geometry, at a predetermined distance from the lens of the microscope and, therefore, the plate is displaced by a known amount so that a processor, which receives the images from the video cameras and the information on the displacement of the plate 35, works out the roto-translation matrices which allow the three coordinates in space of the images detected by the video cameras to be worked out.

21. Device (1) and procedure for computer-assisted microsurgery as described and claimed.
